# EUROPEAN PATENT SPECIFICATION

(11) **EP 0 508 371 B1**
(45) Date of publication and mention of the grant of the patent: **11.02.1998**
(21) Application number: 92105990.3
(22) Date of filing: 07.04.1992
(51) Int. Cl.: C07K 1/14

(54) **Isolation and purification of lantibiotics**
Isolierung und Reinigung von Lantibiotika
Isolation et purification de lantibiotiques

(30) Priority: 11.04.1991 GB 9107717
(43) Date of publication of application: 14.10.1992
(73) Proprietor: Dr. Karl Thomae GmbH, D-88397 Biberach (DE)
(72) Inventor: Allgaier, Hermann, Dr., W-7951 Mittlebiberach (DE); Hentschel, Norbert, W-7950 Biberach 1 (DE); Walter, Joachim, Dr., W-7951 Mittelbiberach (DE); Werner, Rolf-Günter, Prof. Dr., W-7950 Biberach 1 (DE)
(74) Representative: Laudien, Dieter, Dr.

(56) References cited:
- CHEMICAL ABSTRACTS, vol. 110, no. 23, June 5, 1989, Columbus, Ohio, USA H.P. FIEDLER et al. "Purifi- cation of the hydrophilic antibiotics epidermin, gallidermin, and nikkomycin Z by preparative reversed- -phase HPLC" page 583, column 1, abstract-no. 210 845v
- CHEMICAL ABSTRACTS, vol. 111, no. 17, October 23, 1989, Columbus, Ohio, USA P. J. BAKER et al. "Chromato- graphic purification of fermentation products" page 576, column 2, abstract-no. 152 142w
- CHEMICAL ABSTRACTS, vol. 111, no. 13, September 25, 1989, Columbus, Ohio, USA
- S. ISHIDA et al. "Scale-up of hydrophobic interaction chromatography for purifica- tion of antitumor antibiotic SN-07" page 534, column 1, abstract-no. 113 664y
- CHEMICAL ABSTRACTS, vol. 100, no. 9, February 27, 1984, Columbus, Ohio, USA KUSANO HIROSHI "Adsorbent in bioindustry. Application of synthetic absorbent for bioindustry" page 269, column 2, abstract-no. 64 245z
- CHEMICAL ABSTRACTS, vol. 95, no. 26, December 28, 1981, Columbus, Ohio, USA HO PING LUM et al. "Synthesis and application of the new macroreticular adsorbents - the H series of adsorption resins" page 375, column 2, abstract-no. 225 612n
- CHEMICAL ABSTRACTS, vol. 83, no. 19, November 10, 1975, Columbus, Ohio, USA SEAN C. O'CONNOR "Macrore- ticular resin chromatography of antibiotics" page 393, column 2, abstract-no. 162 165y

## Description

This invention relates to the purification of antibiotic compounds of the class known as lantibiotics.

Lantibiotics are a class of small peptide antibiotics characterised by the presence of lanthionine and 3-methyllanthionine bridges. Members of this class include subtilin, nisin, epidermin, gallidermin, pep5, ancovenin, Ro 09-0198, cinnamycin and duramycin.

Lantibiotics can be produced by cultivation of the various strains of micro-organism such as the following:
Pep5 (Staphylococcus epidermis strain 5), epidermin (Staphylococcus epidermin DSM 3095), nisin (Streptococcus lactis, lancefield, group N), gallidermin (Staphylococcus gallinarium DSM 4616), subtilin (Bacillus subtilis NRRL 8-543), ancovenin (Streptomyces sp. no. A647P-Z), Ro 09-0198 (Streptoverticillum griseoverticillatum), duramycin (Streptomyces cinnamoneus forma azacoluta), cinnamycin (Streptomyces cinnamoneus).
A useful description of the foregoing lantibiotics together with literature references therefor can be found in Kellner et al., Eur. J. Biochem. 177: 53 - 59 (1988).

Upon cultivation of the microorganism strain, e.g. by conventional methods, the lantibiotic is secreted into the culture medium. Fermentation can be carried out as described in EP-A-350810.

Known methods of purification include, for example, adsorption on adsorber resins followed by purification with other chromatographic methods.
Thus for example Kellner et al. (supra) discloses a process for the isolation of gallidermin following a 24 hour cultivation of Staphylococcus gallinarum (20 l). This was achieved by first adding Amberlite® XAD-1180 directly to the fermentation vessel in three batches in amounts of 2%, 1% and again 1% by volume of the fermentation broth, which usually contains about 100 mg/l gallidermin. The lantibiotic can be bound in a batch mode to the adsorber resin. The resin was then filtered off and washed with water, then eluted with methanol/0.01 M HCl (9 : 1 by volume) and the active eluate concentrated to a dry mass. After dissolution, the residue was applied to Amberlite® IRC-50 (H⁺-form) at pH 5.5, the ion exchanger washed with water and gallidermin eluted with 0.1 M HCl. After desalting via adsorption, again with Amberlite® XAD-1180, lyophilisation yielded 1.0 g of crude lantibiotic. After final purification by reversed phase-HPLC with Nucleosil® SC18 - 300 as stationary phase and various gradients of acetonitrile/0.1% trifluoroacetic acid in water, 100 mg of pure gallidermin were obtained. With about 2000 mg gallidermin in the fermentation broth at the end of the cultivation, this 100 mg yield at the end of purification corresponds to a relative overall yield of 5%.

Hörner et al., in Appl. Microbiol. Biotechnol. 30, 219 - 225 (1989), describe a method for the fermentation and isolation of epidermin. Purification is carried out essentially similarly to that described above for gallidermin; namely initial adsorption on Amberlite® XAD-1180 followed by elution, application to the weak cation exchanger Amberlite® IRC-50 and finally purification by preparative HPLC on Nucleosil® 100 C-18. A purity of 80% is reported for this combined use of adsorption and ion exchange chromatography.

For commercial use of a lantibiotic it is apparent that a higher purity is required, and moreover improved yields for the purification procedures are also desirable.
The difficulties of purification of lantibiotics are related to the biological syntheses and chemical structures of these biologically active peptides and include the following:
mass production of up to 1 g/l of a lantibiotic can only be achieved with fermentation media containing meat extract or malt extract, which results in complex mixtures for subsequent downstream processing;
due to their high content of basic and hydrophobic amino acids only a few carefully selected isolation techniques can be applied;
the product instability at higher pH-values significantly limits the range of buffer systems which can be used; and
the occurrence of biologically active oligomers and monomers requires the use of high resolution separation techniques.

Accordingly, an object of the present invention is to provide an improved method for the purification of lantibiotics.

Thus viewed from one aspect the present invention provides a process for lantibiotic purification comprising obtaining a lantibiotic containing fermentation medium and subjecting said medium or lantibiotic containing media deriving therefrom to successive steps of:
adsorption of the lantibiotic out of the fermentation broth on a styrene-divinyl-copolymerisate matrix such as Amberlite® XAD-1180 for example;
elution of the lantibiotic from the styrene-divinyl-copolymerisate matrix using a methanolic solution containing from 0.001 M to 0.1 M of hydrochloric acid;
subjecting the eluate to cation exchange chromatography using a strong cation exchange type with a polysaccharide based matrix;
elution using a methanolic aqueous buffer containing 10 to 40% (by volume) of methanol and 0.5 to 1.5 M of sodium chloride or potassium chloride at a pH-value of 1 to 3;
subjecting this eluate to hydrophobic interaction chromatography, except for reversed phase HPLC, using a polysaccharide based matrix coupled with alkanes of different chain length as ligands;
elution using an aqueous methanolic solution containing 10 to 30% (by volume) of methanol and up to 0.02 M of sodium phosphate or potassium phosphate at a pH-value from 5.0 to 6.0;
subjecting the eluate to anion exchange chromatography using a weak anion exchange type with a polysaccharide based matrix;
desalting the solution which flows through by ultra- and/or diafiltration; and
if desired, subsequent lyophilisation.
In the process of the invention it is particularly preferred to use the following matrices/chromatographic media:
as a styrene-divinyl-copolymerisate-Amberlite® XAD-1180;
as a strong cation exchanger-S-Sepharose® FF;
as a polysaccharide-based alkane-coupled matrix - Octyl-Sepharose® CL 4B; and
as a weak anion exchanger - DEAE-Sepharose® FF.

The process of the invention is particularly advantageous as it enables the following purification/extraction targets to be achieved:
an overall product purity greater than 99%;
the ability to purify large amounts of peptides on pilot and production-scales; and
an overall yield of at least about 50%. It also enables one to obtain a first indication of production costs for commercially marketed material.

Particularly preferred embodiments of the individual steps of the process of the invention will now be described in further detail.

After fermentation, the broth is adjusted to a pH of between 4.5 and 6.5, preferably to a pH of 5.5, with 0.1 M HCl. Three batches of Amberlite® XAD 1180 in amounts of 2%, 1% and again 1% by volume of the fermentation broth at intervals of between 30 and 60 minutes are added to the fermentation broth whilst stirring.
After adsorption of the lantibiotic, the resin is filtered off, washed with water and filled into a column. The resin is then washed with methanol/water 1:1 (by volume) to remove unspecifically bound substances from the column. After washing, the column is eluted with a mixture of 90% methanol and 10% 0.01 M to 1 M HCl (by volume) as eluant, preferably a solution of 90% methanol and 10% 0.01 M HCl (by volume).

In the above mentioned XAD-1180 adsorption step, the organisms are removed from the resin by washing extensively with water. Alternatively they can be removed by microfiltration.
The subsequent adsorption, washing and desorption steps are then all carried out in a column mode.

The cation exchange chromatography is preferably carried out using a strong cation exchange type with a polysaccharide based matrix to prevent unwanted hydrophobic interactions with the matrix during the elution mode. The preferred material for the cation exchange chromatography is a material such as S-Sepharose® Fast Flow or its equivalent.
The S-Sepharose® column should be equilibrated before use with a solution containing, for example, sodium phosphate, e.g. with a 0.3 to 0.8 M sodium phosphate solution. After loading of the S-Sepharose® column with the XAD-1180 eluate, the pH is adjusted to between 3.5 and 5.0 with sodium hydroxide solution. The column is then washed with a buffer solution containing between 0.4 and 1.0 M sodium chloride per litre of water. The subsequent elution can be carried out with a suitable buffer, for example with a buffer composed of between 0.5 and 1.5 M sodium or potassium chloride, 10 to 40% (by volume) methanol and a pH of 1 to 3 adjusted with a 1 M hydrochloric acid solution. Preferably a buffer composed of 0.9 M sodium chloride, containing 30% methanol (by volume) adjusted to a pH of 2 is used. Linear flow rates of 75 cm/h for the loading, washing and elution modes are preferably used for the S-Sepharose® FF step.
Due to the high content of sodium or potassium chloride in the S-Sepharose® pool it can favourably be applied to subsequent hydrophobic interaction chromatography.
The hydrophobic interaction chromatography can suitably be carried out using a polysaccharide based matrix coupled with alkanes of different chain length as ligands. Octyl Sepharose® CL-4B is the preferred material as stationary phase.

In a preferred embodiment a lantibiotic solution subjected to hydrophobic interaction chromatography contains a salt such as sodium chloride, and the column is previously equilibrated with a buffer containing the same salt.

For example the collected lantibiotic containing fractions from the cation exchange chromatography phase may be adjusted to contain between 0.9 and 1.5 M of a sodium or potassium salt, such as sodium chloride, per litre by the addition of crystalline sodium or potassium salts. A suitable pH range for processing by hydrophobic interaction chromatography is pH 4.5 to 6.0. Adjustment of the pH can conveniently be made using sodium hydroxide solution. Before loading of the column the methanol concentration must be adjusted down to 10% (by volume) by the addition of water.

The stationary phase for the hydrophobic interaction chromatography is preferably equilibrated by an aqueous buffer comprising for example sodium or potassium chloride (0.9 to 1.8 M) and sodium phosphate (0.0075 to 0.015 M) . The control of process parameters such as flow rates can be suitably adjusted within a certain range. A suitable eluant is composed of 70 to 90% (by volume) of water, 10 to 30% (by volume) of methanol and contains 0 to 0.02 M of a sodium or potassium salt, such as sodium phosphate, pH range 5.0 to 6.0.

A buffer composed of 0.01 M sodium phosphate, 10% methanol (by volume) with a pH-value of pH 5.5 is preferably used.
The advantages of hydrophobic interaction chromatography compared with reversed phase high performance liquid chromatography (RP-HPLC) are a higher capacity, and the use of less organic solvents. Capacity in this regard means the amount of lantibiotic which can be bound to a certain quantity of chromatography material.
Even though the purity of the lantibiotics after the hydrophobic interaction chromatography is higher than 99% (assessed by HPLC at 210 nm) a weak anion exchange chromatography step, using for example DEAE Sepharose® FF, for the removal of slightly yellow coloured substances is most preferably used.
The column is equilibrated with the Octyl-Sepharose® elution buffer adjusted to a pH of 7. The Octyl-Sepharose®; eluant is adjusted to pH 7 with an aqueous 1 M sodium hydroxide solution. The lantibiotics are passed through the column and washed out with the equilibration buffer, leaving the coloured substances bound to the anion exchange material.

The eluates from the chromatographic steps are desalted before further processing; this is usually done after the anion exchange chromatography step due to the low ionic strength.

Suitable desalting techniques are well known in the art, such as gel chromatography or ultra- and/or diafiltration.
In the case of gallidermin, for example, the eluate of the anion exchange chromatography step may be concentrated to a gallidermin concentration of 1 to 10 mg/ml and repeatedly dialyzed with distilled water, for example, up to 25 times, preferably using a membrane having a cut-off of 1 KD (Kilo-Dalton) before lyophilisation. A membrane such as Filtron Omega 1 KD membrane or equivalent can be used. The lyophilisation of the final product is carried out according to conventional methods.

It is envisaged that the process of the invention is suitable for purifying not only epidermin and gallidermin, but also other lantibiotics including
nisin, subtilin, duramycin, ancovenin, cinnamycin, Pep5 and Ro 09-0198.

Preferred embodiments of the invention will now be described by way of example and with reference to the accompanying drawings in which:

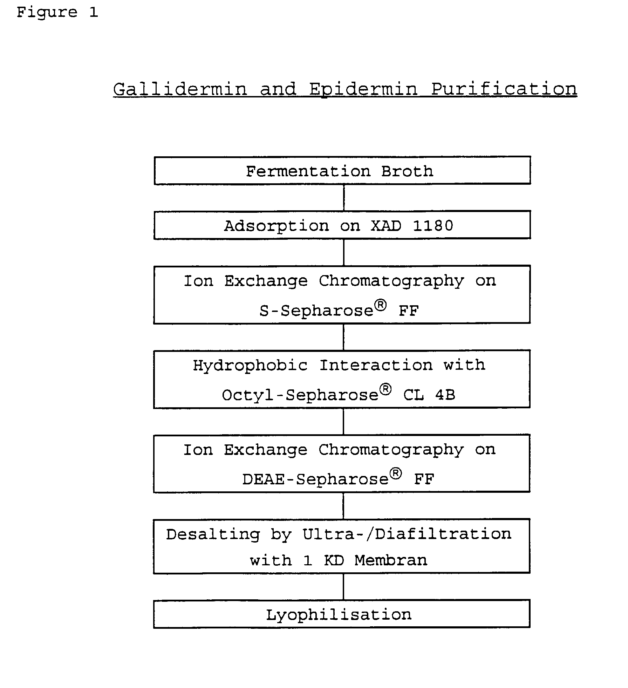

Figure 1 summarizes the described purification scheme for the purification of lantibiotics; and
Figures 2 to 7 are chromatograms indicative of product purity at various stages of the process.

In the following examples, all given ratios and percentages are by weight, unless otherwise specified.

### Example 1

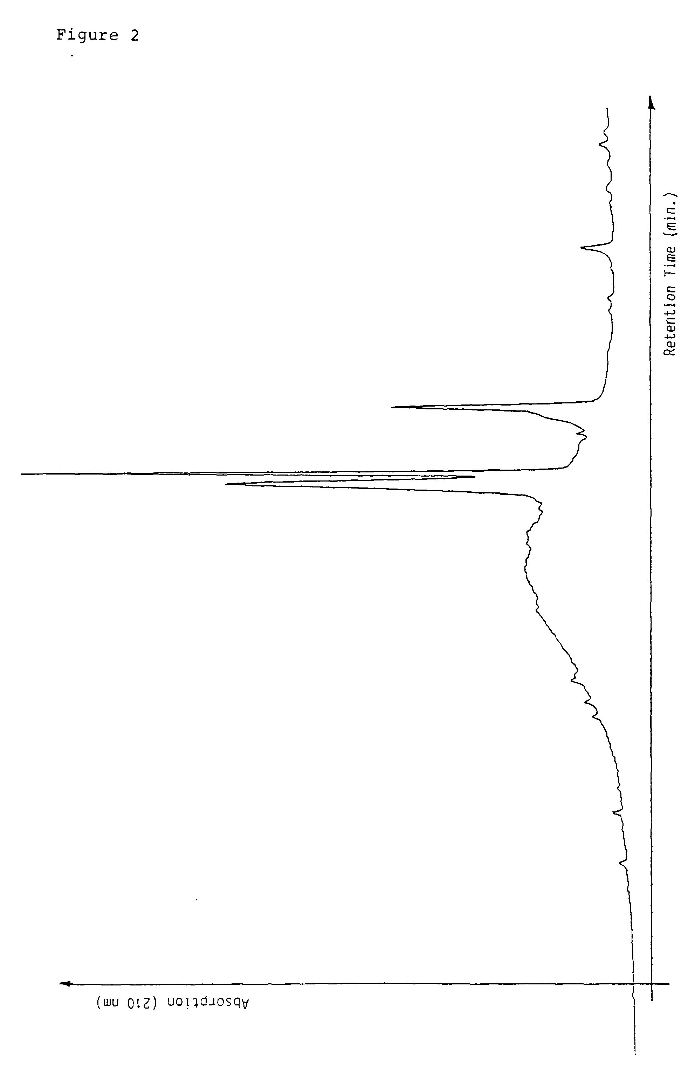

A gallidermin containing culture medium was prepared using the procedure described in the section headed "Fermentation" of Example 1 of EP-A-342486.
After the accumulation of 119.8 mg/l of gallidermin in the fermentation broth (the concentration being determined by periodic HPLC-measurements) Amberlite® XAD-1180 was added directly to the fermentation broth after adjusting the broth to pH 5.5 (HCl) in two batches, each 2% by volume, at intervals of 45 minutes. The adsorption of the antibiotic was measured by the disappearance of the antibiotic from the fermentation broth.
The resin was then filtered off, washed with water and then filled into a chromatographic column (h = 77 cm, d = 10 cm, v = 6050 ml) for further processing. Applying a linear flow rate of 385 cm/h, the column was washed with water/methanol (1:1 by volume) for a total volume of 5 column volumes, then the elution was carried out with a buffer composed of methanol/0.01 M HCl (9:1 by volume) and a flow rate of 231 cm/h. Fractions of 1 CV were taken and pooled according to continuous UV-detection at 254 nm, thin layer chromatography and high performance liquid chromatography. Fractions 1 to 7 were pooled to give a total volume of 41 1 containing 279 mg/l gallidermin. The step yield was thus calculated to be 91%. An HPLC-chromatogram of the pooled and homogenized fractions is shown in Fig. 2.
The eluate from the XAD-1180 column was adjusted to pH 4.0 with 1.0 M sodium hydroxide, then applied to a S-Sepharose® Fast Flow column (h = 13.0 cm, d = 14 cm, v = 2000 ml) equilibrated with a buffer composed of 0.3 M sodium phosphate pH 4.0 (two column volumes) using a linear flow rate of 150 cm/h.

After loading, the column was washed with two column volumes of 0.5 M NaCl and eluted with a buffer composed of 0.9 M NaCl, 0.01 M HCl with 30% methanol (by volume) at a linear flow rate of 75 cm/h.

Fractions of approximately one column volume were assayed for gallidermin and pooled according to continuous UV-detection at 254 nm and HPLC. Fractions 1 to 4 gave a total volume of 7140 ml containing 1338 mg/l gallidermin measured by HPLC and resulting in a step yield of 83.4%.

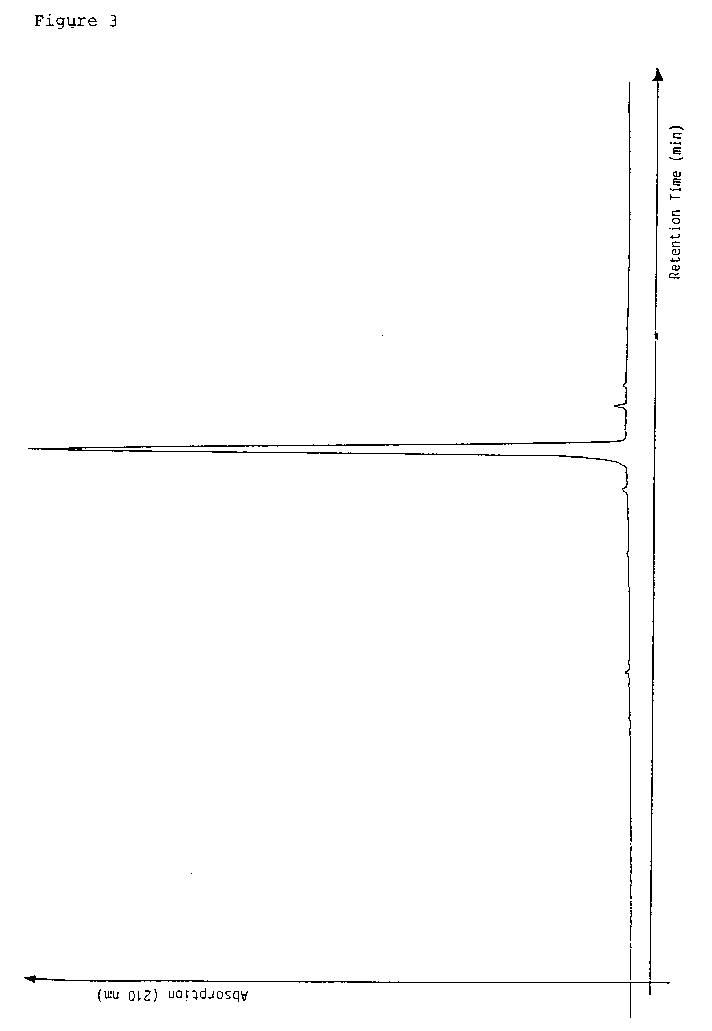

A representative HPLC-chromatogram of the pooled and homogenized fractions is shown in Fig. 3.

The S-Sepharose® pool was diluted from 30 to 10% methanol and then adjusted to pH 5.5 with 1 M sodium hydroxide and with solid NaCl to 1.2 M NaCl.

An Octyl-Sepharose® column (h = 11.7 cm, d = 18 cm, V = 2977 ml) was prepared and equilibrated with an aqueous buffer composed of 1.2 M sodium chloride and 0.01 M sodium phosphate.
Four column volumes at a linear flow rate of 60 cm/h were used. The adjusted S-Sepharose® pool was loaded to the equilibrated column at a flow rate of 60 cm/h followed by a two column volume wash with the equilibration buffer.
Elution was performed with a buffer containing 0.01 M sodium phosphate with 10% (by volume) methanol at pH 5.5. Gallidermin containing fractions were pooled to yield a total volume of 7590 ml. From the gallidermin content a step yield of 78.1% was calculated.

The DEAE-Sepharose® column was equilibrated with the Octyl-Sepharose® elution buffer adjusted to pH 7.0.
The eluate from the Octyl-Sepharose® column was adjusted to pH 7.0 with a 0.1 M sodium hydroxide solution and then loaded onto the DEAE-Sepharose® column. After loading, the gallidermin was washed out with the equilibration buffer. For this step a yield of 98.7% was calculated.
The DEAE-Sepharose® pool was concentrated to approximately 5 mg/ml and diafiltered 20 fold with distilled water using a membrane having a cut-off of 1 KD. The lyophilized diafiltrate gave a yield of 6.0 g of gallidermin.

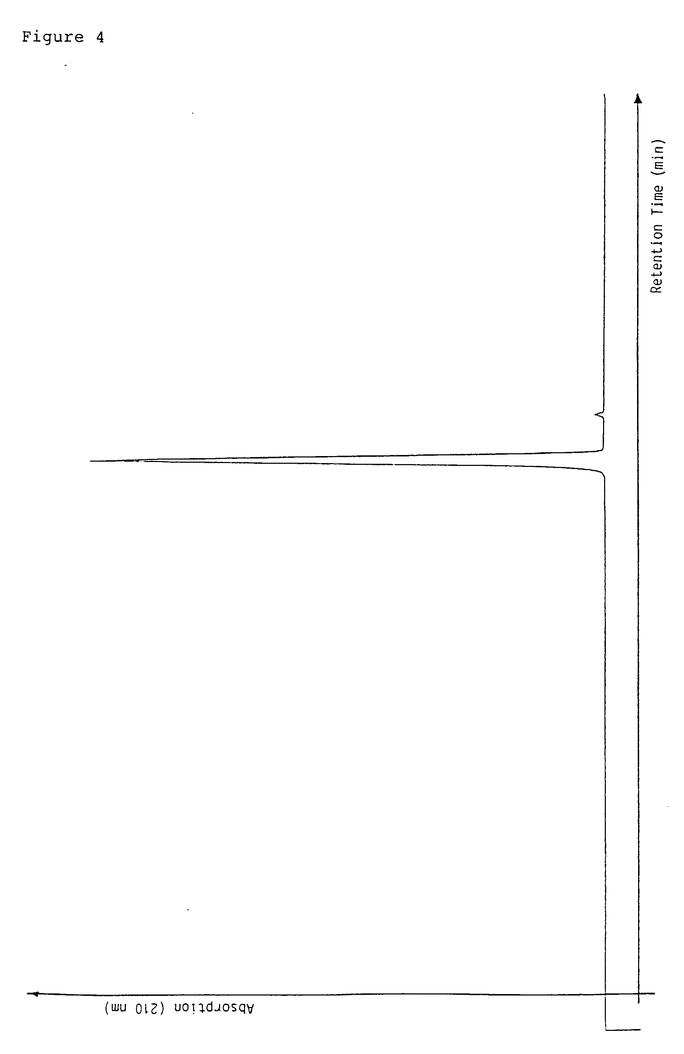

A chromatogram of the pooled and homogenized fractions is shown in Fig. 4.

The increase in the product purity, measured by analytical reversed phase HPLC (210 nm), is shown in Figure 2 (XAD 1180 pool), Figure 3 (S-Sepharose® FF pool) and Figure 4 (final product).

The purity of the final gallidermin product was assessed by reversed phase high performance liquid chromatography and quantitative amino acid analyses. The content of gallidermin assessed by RP-HPLC was 99.3% and by quantitative amino acid analysis was 99.1%.

### Example 2

An Epidermin-containing culture medium was prepared using the procedure described in the section headed "Fermentation" of Example 1 of EP-A-342486.
After the accumulation of 72.7 mg/l of Epidermin in the fermentation broth (the concentration being determined by periodic HPLC-measurements) Amberlite® XAD-1180 was added directly to the fermentation broth after adjusting the broth at pH 5.5 (HCl) in two batches, each 2% by volume, at intervals of 45 minutes. The adsorption of the antibiotic was measured by the disappearance of the antibiotic from the fermentation broth.

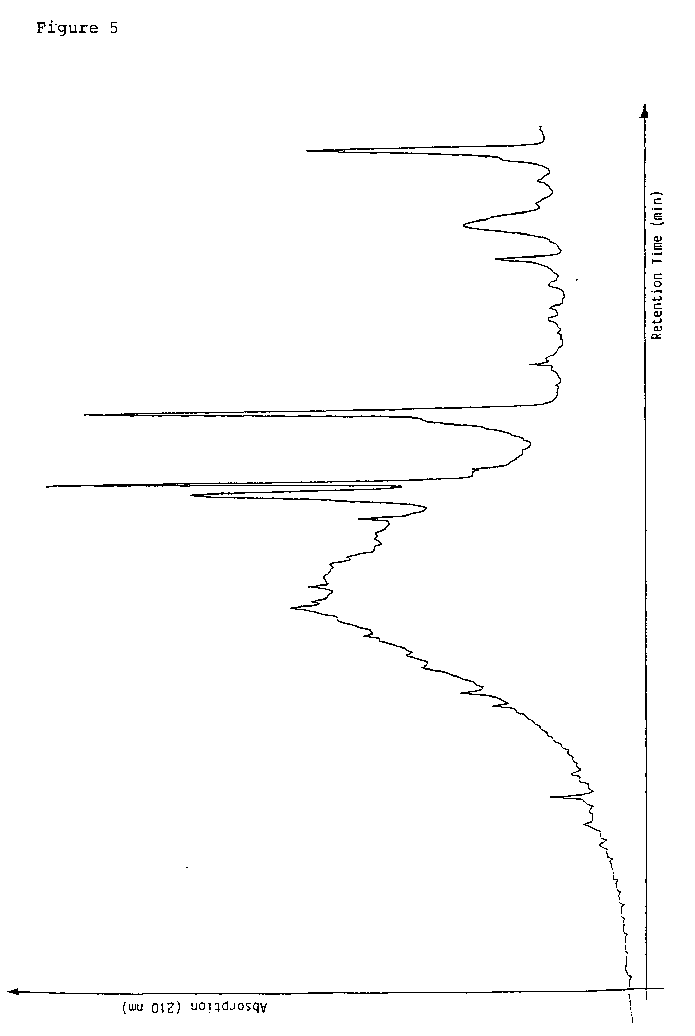

The resin was then filtered off, washed with water and then filled into a chromotographic column (h = 10 cm, d = 12 cm, v = 1130 ml) for further processing. Applying a linear flow rate of 50 cm/h, the column was washed with water/methanol (1:1 by volume) for a total volume of 5 column volumes (CV), then the elution was carried out with a buffer composed of methanol/0.01 M HCl (9:1 by volume) and a flow rate of 30 cm/h. Fractions of 1 CV were taken and pooled according to continuous UV-detection at 254 nm, thin layer chromatography and high performance liquid chromatography. Fractions 1 to 8 were pooled to give a total volume of 10 l containing 200 mg/l Epidermin. The step yield was thus calculated to be 89%. An HPLC-chromatogram of the pooled and homogenized fractions is shown in Fig. 5.

The eluate from the XAD-1180 column was adjusted to pH 4.0 with 1.0 M sodium hydroxide, then applied to a S-Sepharose® Fast Flow column (h = 21.5 cm, d = 2.6 cm, v = 114 ml) equilibrated with a buffer composed of 0.3 M sodium phosphate pH 4.0 (two column volumes) and a linear flow rate of 150 cm/h.

After loading, the column was washed with two column volumes of 0.5 M NaCl and eluted with a buffer composed of 0.9 M NaCl, 0.01 M HCl with 30% methanol (by volume) at a linear flow rate of 75 cm/h.

Fractions of approximately one column volume were assayed for epidermin and pooled accordingly to continuous UV-detection at 254 nm and HPLC. Fractions 1 to 7 gave a total volume of 1150 ml containing 1412 mg/l Epidermin measured by HPLC and resulting in a step yield of 81.2%.

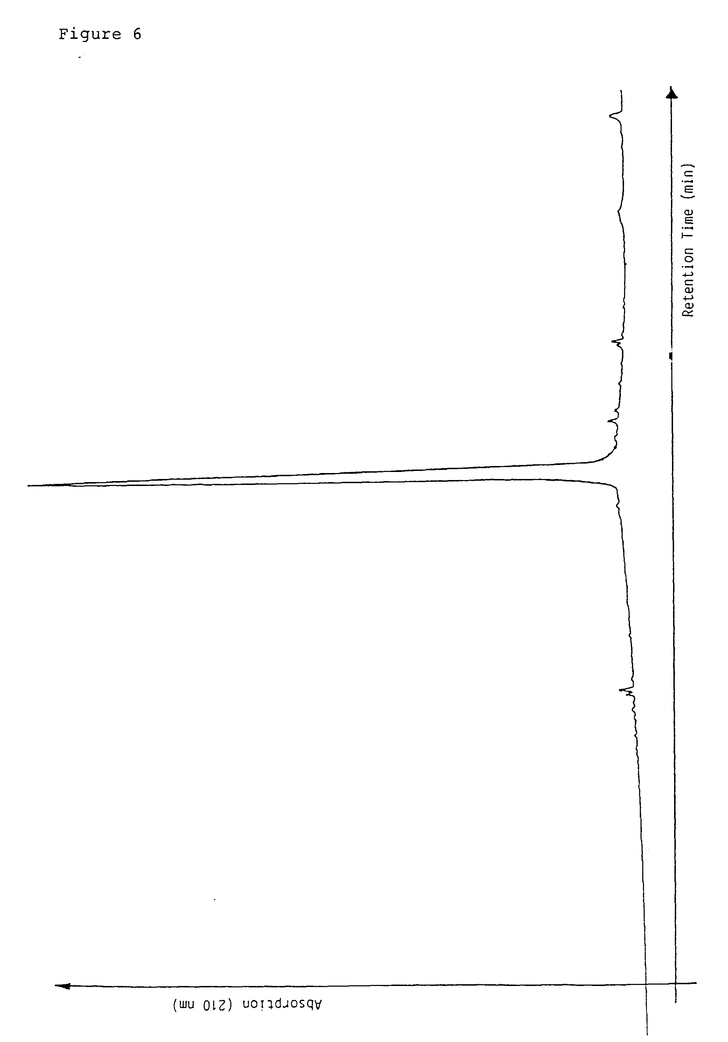

An HPLC-chromatogram of the pooled and homogenized fractions is shown in Fig. 6.

The S-Sepharose® pool was diluted from 30 to 10% (by volume) methanol and then adjusted to pH 5.5 with 1 M sodium hydroxide and with solid NaCl to 1.2 M NaCl.

An Octyl-Sepharose® column (h = 12.7 cm, d = 5 cm, V = 249 ml) was prepared and equilibrated with an aqueous buffer composed of 1.2 M sodium chloride and 0.01 M sodium phosphate. Four column volumes at a linear flow rate of 60 cm/h were used. The adjusted S-Sepharose® pool was loaded to the equilibrated column at a flow rate of 60 cm/h followed by a two column volume wash with the equilibration buffer.

Elution was performed with a buffer containing 0.01 M sodium phosphate with 10% (by volume) methanol at pH 5.5. Epidermin containing fractions were pooled to yield a total volume of 1050 ml. From the gallidermin content one can calculate a step yield of 75.2%.

The DEAE-Sepharose® column was equilibrated with the Octyl-Sepharose® elution buffer adjusted to pH 7.0.

The eluate from the Octyl-Sepharose® column was adjusted to pH 7.0 with a 0.1 M sodium hydroxide solution and then loaded onto the DEAE-Sepharose® column. After loading the epidermin was washed out with the equilibration buffer. For this step a yield of 98.7% was calculated.

The Sepharose® pool was concentrated to approximately 5 mg/ml and diafiltered 20 fold with distilled water using a membrane having a cut-off of 1 KD. The lyophilized diafiltrate gave a yield of 1.0 g of epidermin.

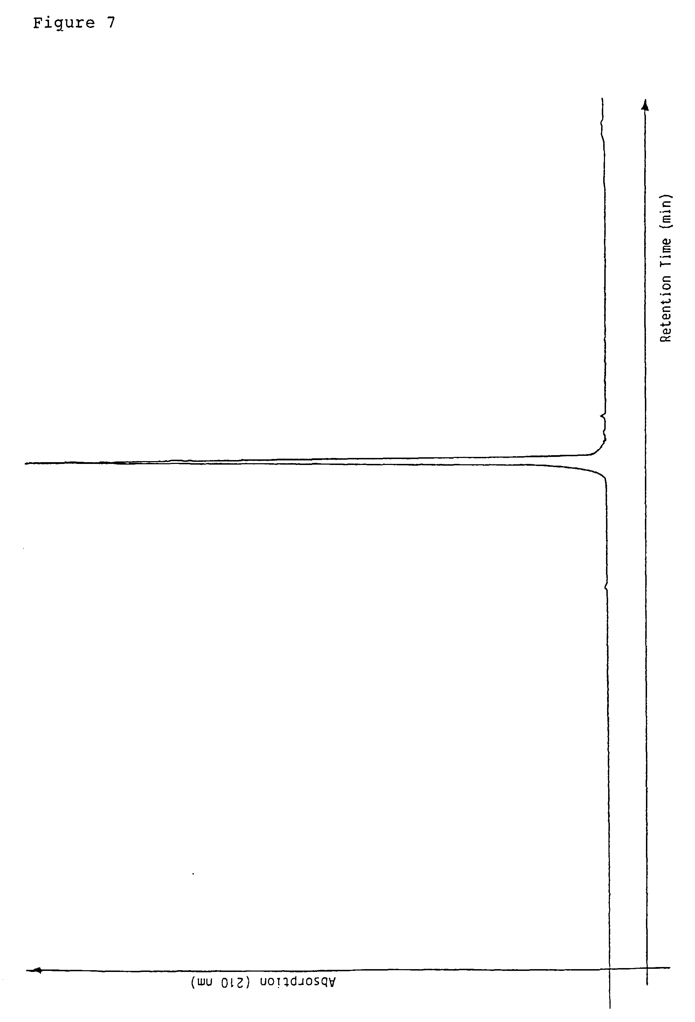

A chromatogram of the pooled and homogenized fractions is shown in Fig. 7.

The increase in the product purity, measured by analytical reversed phase HPLC (210 nm), is shown in Figure 5 (XAD 1180 pool), Figure 6 (S-Sepharose® FF pool) and Figure 7 (final product).
The purity of the final epidermin product was assessed by reversed phase high performance liquid chromatography and quantitative amino acid analyses. The content of epidermin assessed by RP-HPLC was 99.1% and by quantitive amino acid analysis was 99.1%.

Table 1 below gives an overview of the overall yields of gallidermin and epidermin as purified in Examples 1 and 2 respectively. It shows the surprisingly high overall yields of the pure products, which are about 50% (by weight) in each case.

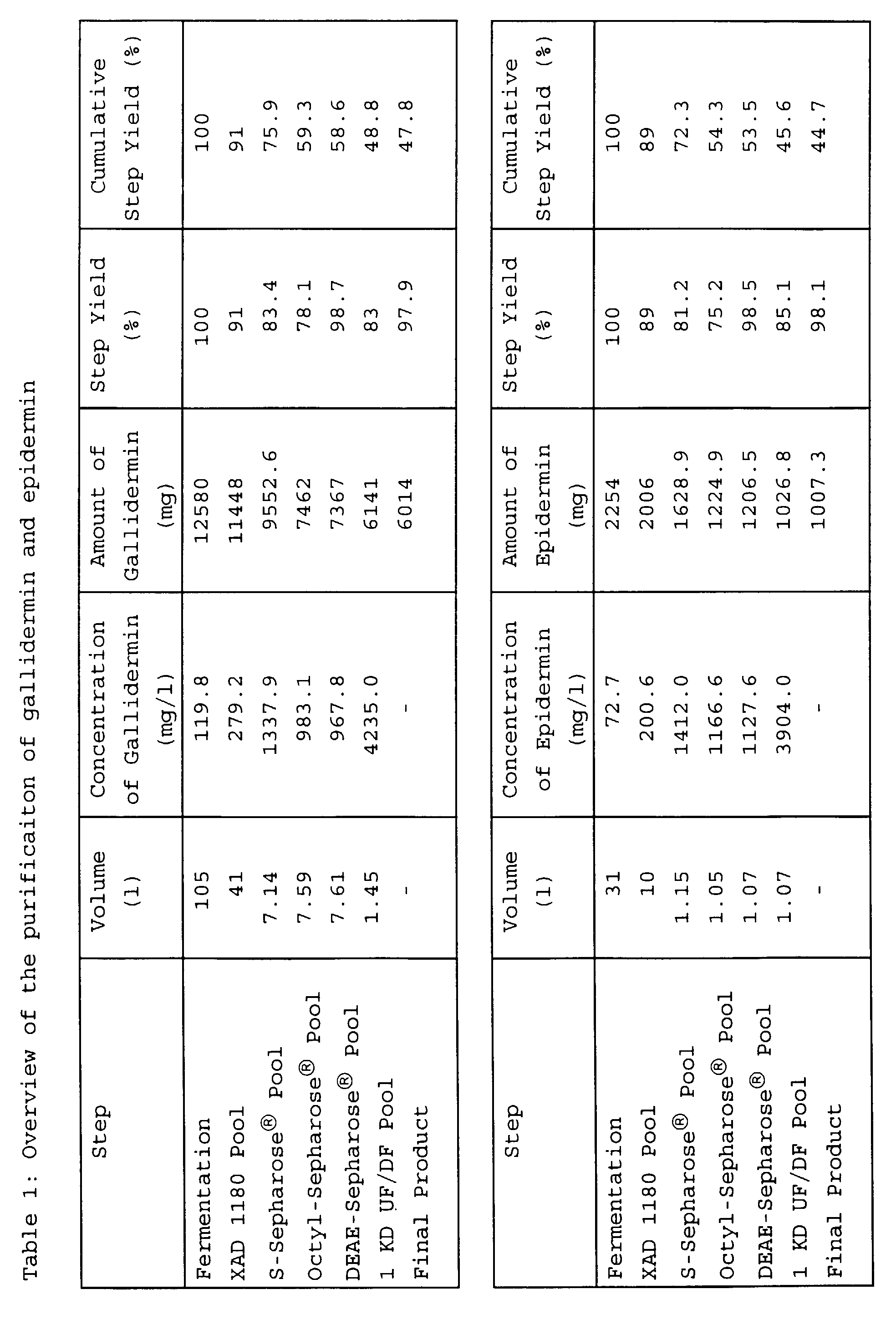

## Claims

1. A process for lantibiotic purification providing an overall yield of at least about 50% and an overall product purity greater than 99% comprising obtaining a lantibiotic containing fermentation medium and subjecting said medium or lantibiotic containing media deriving therefrom to successive steps of:
adsorption of the lantibiotic out of the fermentation broth on a styrene-divinyl-copolymerisate matrix;
elution of the lantibiotic from the styrene-divinyl-copolymerisate matrix using a methanolic solution containing from 0.001 M to 0.1 M of hydrochloric acid;
subjecting the eluate to cation exchange type chromatography using a strong cation exchange type with a polysaccharide based matrix;
elution using a methanolic aqueous buffer containing 10 to 40% (by volume) of methanol and 0.5 to 1.5 M of sodium chloride or potassium chloride at a pH-value of 1 to 3;
subjecting this eluate to hydrophobic interaction chromatography, except for reversed-phase HPLC, using a polysaccharide based matrix coupled with alkanes of different chain length as ligands;
elution using an aqueous methanolic solution containing 10 to 30% (by volume) of methanol and up to 0.02 M of sodium phosphate or potassium phosphate at a pH-value from 5.0 to 6.0;
subjecting the eluate to anion exchange chromatography using a weak anion exchange type with a polysaccharide based matrix;
desalting the solution which flows through by ultra- and/or diafiltration; and if desired, subsequent lyophilisation.

2. A process as claimed in claim 1 wherein there is used
as a styrene-divinyl-copolymerisate-Amberlite® XAD-1180;
as a strong cation exchanger-S-Sepharose® FF;
as a polysaccharide based alkane-coupled matrix Octyl-Sepharose® CL 4B;
and as a weak anion exchanger - DEAE-Sepharose® FF.

3. A process as claimed in any one of claims 1 or 2 wherein desalting is effected by ultra- and/or diafiltration using a membrane with a cut-off of 1 kilo Dalton.

4. The process of claim 1 for purification of a lantibiotic selected from epidermin, gallidermin, nisin, subtilin, duramycin, ancovenin, cinnamycin, Pep5 and Ro 09-0198.

5. The process of claim 1 for purification of a lantibiotic selected from gallidermin and epidermin.

## Patentansprüche

1. Verfahren zur Reinigung von Lantibiotikum unter Gewährleistung einer Gesamtausbeute von mindestens etwa 50 % und einer Gesamt-Produktreinheit von mehr als 99 %, umfassend die Gewinnung eines Lantibiotikum enthaltenden Fermentationsmediums und das Unterwerfen des genannten Mediums oder davon abgeleiteter, Lantibiotikum enthaltender Medien einer Aufeinanderfolge von Schritten von:
Adsorption des Lantibiotikums aus der Fermentationsbrühe heraus auf eine Styrol-Divinyl-Copolymerisat-Matrix;
Elution des Lantibiotikums aus der Styrol-Divinyl-Copolymerisat-Matrix unter Verwendung einer 0,001 M bis 0,1 M Salzsäure enthaltenden methanolischen Lösung;
Unterwerfung des Eluats einer Chromatographie des Kationenaustauscher-Typus unter Verwendung eines starken Kationenaustauscher-Typus mit einer Matrix auf Basis von Polysaccharid;
Elution unter Verwendung eines 10 bis 40 Vol.-% Methanol und 0,5 bis 1,5 M Natriumchlorid oder Kaliumchlorid bei einem pH-Wert von 1 bis 3 enthaltenden methanolischen wässrigen Puffers;
Unterwerfung dieses Eluats einer Hydrophobchromatographie, ausgenommen die Umkehrphasen-HPLC, unter Verwendung einer mit Alkanen verschiedener Kettenlängen als Liganden gekuppelten Matrix auf Basis von Polysaccharid;
Elution unter Verwendung einer 10 bis 30 Vol.-% Methanol und bis zu 0,02 M Natriumphosphat oder Kaliumphosphat bei einem pH-Wert von 5,0 bis 6,0 enthaltenden wässrigen methanolischen Lösung;
Unterwerfung des Eluats einer Anionenaustauscherchromatographie unter Verwendung eines schwachen Anionenaustauscher-Typus mit einer Matrix auf Basis von Polysaccharid;
Entsalzung der durchfliessenden Lösung durch Ultra- und/oder Diafiltration; und falls erwünscht, nachfolgende Gefriertrocknung.

2. Verfahren nach Anspruch 1, bei welchem verwendet wird:
als Styrol-Divinyl-Copolymerisat Amberlite^{(R)} XAD-1180;
als starker Kationenaustauscher S-Sepharose^{(R)} FF;
als mit Alkan gekuppelte Matrix auf Basis von Polysaccharid Octyl-Sepharose^{(R)} CL 4B;
und als schwacher Anionenaustauscher DEAE-Sepharose^{(R)} FF.

3. Verfahren nach einem der Ansprüche 1 oder 2, bei welchem die Entsalzung durch Ultra- und/oder Diafiltration unter Verwendung einer Membran mit einem Grenzrückhaltevermögen bei 1 kiloDalton erfolgt.

4. Verfahren nach Anspruch 1 zur Reinigung eines unter Epidermin, Gallidermin, Nisin, Subtilin, Duramycin, Ancovenin, Cinnamycin, Pep5 und Ro 09-0198 ausgewählten Lantibiotikums.

5. Verfahren nach Anspruch 1 zur Reinigung eines unter Gallidermin und Epidermin ausgewählten Lantibiotikums.

## Revendications

1. Procédé de purification de lantibiotique procurant un rendement global d'au moins environ 50% et une pureté globale du produit supérieure à 99% comprenant l'obtention d'un milieu de fermentation contenant un lantibiotique et la soumission dudit milieu ou . de milieux contenant un lantibiotique qui en dérivent à des étapes successives de: adsorption du lantibiotique à partir du bouillon de fermentation sur une matrice de copolymère styrène-divinylique;
élution du lantibiotique de la matrice de copolymère styrène-divinylique à l'aide d'une solution méthanolique contenant de 0,001 M à 0,1 M d'acide chlorhydrique;
soumission de l'éluat à une chromatographie de type échange de cations à l'aide d'un type d'échangeur de cations fort avec une matrice à base de polysaccharide;
élution à l'aide d'un tampon aqueux méthanolique contenant 10 à 40% (en volume) de méthanol et 0,5 à 1,5 M de chlorure de sodium ou de chlorure de potassium à un pH de 1 à 3;
soumission de cet éluat à une chromatographie d'interaction hydrophobe, à l'exception de la CLHP en phase inverse, à l'aide d'une matrice à base de polysaccharide couplée à des alcanes de longueur de chaîne différente comme ligands;
élution à l'aide d'une solution aqueuse méthanolique contenant 10 à 30% (en volume) de méthanol et jusqu'à 0,02 M de phosphate de sodium ou de phosphate de potassium à un pH de 5,0 à 6,0;
soumission de l'éluat à une chromatographie d'échange d'anions à l'aide d'un type d'échangeur d'anions faible avec une matrice à base de polysaccharide;
élimination des sels de la solution qui s'écoule par ultra- et/ou diafiltration; et si on le souhaite, lyophilisation subséquente.

2. Procédé selon la revendication 1 dans lequel on utilise comme copolymère styrène-divinylique Amberlite® XAD-1180;
comme échangeur de cations fort S-Sepharose® FF;
comme matrice à base de polysaccharide couplée à des alcanes Octyl-Sepharose® CL 4B;
et comme échangeur d'anions faible DEAE-Sepharose® FF.

3. Procédé selon l'une quelconque des revendications 1 et 2 dans lequel l'élimination des sels est accomplie par ultra- et/ou diafiltration à l'aide d'une membrane ayant une coupure de 1 kilodalton.

4. Procédé selon la revendication 1 pour la purification d'un lantibiotique choisi parmi l'épidermine, la gallidermine, la nisine, la subtiline, la duramycine, l'ancovénine, la cinnamycine, Pep5 et Ro 09-0198.

5. Procédé selon la revendication 1 pour la purification d'un lantibiotique choisi parmi la gallidermine et l'épidermine.
